# EUROPEAN PATENT SPECIFICATION

(11) **EP 3 373 891 B1**
(45) Date of publication and mention of the grant of the patent: **31.08.2022**
(21) Application number: 15908084.5
(22) Date of filing: 13.11.2015
(51) Int. Cl.: A61K 8/24, A61K 8/21, A61K 8/19, A61K 8/02, A61K 8/73, A61Q 11/00

(54) **DENTIFRICE COMPOSITIONS WITH IMPROVED FLUORIDE STABILITY**
ZAHNPASTAZUSAMMENSETZUNGEN MIT VERBESSERTER FLUORSTABILITÄT
COMPOSITIONS DE DENTIFRICE PRÉSENTANT UNE MEILLEURE STABILITÉ DU FLUORURE

(43) Date of publication of application: 19.09.2018
(73) Proprietor: The Procter & Gamble Company, Cincinnati, OH 45202 (US)
(72) Inventor: BASA, Swapna, Beijing 101312 (CN); BERTA, James Albert, Cincinnati, Ohio 45202 (US); YANG, Hongmei, Beijing 101312 (CN); STRAND, Ross, Singapore 138547 (SG)
(74) Representative: P&G Patent Germany
(86) International application number: PCT/CN2015/094506
(87) International publication number: WO 2017/079954

(56) References cited:
- EP-A2- 0 092 929
- WO-A1-2009/134657
- WO-A1-2012/057739
- WO-A1-2013/041419
- WO-A1-2014/100928
- WO-A1-2015/172651
- WO-A1-2015/172652
- GB-A- 2 188 548
- ZHAO YINFA ET AL.: 'Y©g?o Yòng T®su?ng® Lìd Pîngji®' TOOTHPASTE INDUSTRY vol. 2007, no. 2, 30 April 2007,

## Description

### FIELD OF THE INVENTION

The present invention relates to dentifrice compositions having high water and high carbonate levels, a fluoride ion source and a thickening system as claimed.

### BACKGROUND OF THE INVENTION

Dentifrice compositions are well known for dental and oral hygiene care. High water (e.g., >44 wt%) and high carbonate (e.g., >24 wt%) formulation chassis are a cost effective for many markets and consumers (WO2009/134657A1, WO2015/172651A1, WO2015/172652). However, this formulation chassis sometimes has fluoride ion stability issues that often exacerbated when there are high temperatures and/or long distribution times such as in some developing markets. Fluoride, and its associated benefits in dentifrice composition, is critical for a user's experience and product acceptance. There is a need to provide such dentifrice formulations having improved fluoride ion stability even in high water formulations.

### SUMMARY OF THE INVENTION

The present invention is based, in part, on the surprising observation that in high water and high carbonate dentifrice formulations, calcium carbonate particles size has an important impact in fluoride stability. Furthermore, this fluoride ion stability effect is further enhanced at pH conditions that are greater than pH 8.0. Particle size distribution can be characterized by conventional D98, D90, D50, or D10 parameters.

Accordingly, an advantage of the present invention is improved soluble fluoride stability over time (in the high water high carbonate dentifrice formulation claimed herein).

One aspect of the invention provides for a dentifrice composition comprising: (a) 45% to 55%, by weight of the composition, of water; (b) 25% to 50%, by weight of the composition, of a calcium carbonate, wherein the calcium carbonate has a particle distribution size of D98 from 35 microns to 46 microns as measured by laser diffraction particle sizing per method ISO 13320-1-1999; (c) 0.0025% to 2%, by weight of the composition, of a fluoride ion source; (d) a thickening system comprising at least a thickening polymer, wherein the thickening polymer is selected from a combination of (i) 0.01% to 3% of a carboxymethyl cellulose (CMC) by weight of the composition; (ii) 0.01% to 2.5% by weight of the composition of a linear sulfated polysaccharide, and; (iii) 0.01% to 7% by weight of the composition of a natural gum, wherein the natural gum is selected from the group consisting of gum karaya, gum arabic, gum tragacanth, xanthan gum and (iv) combination of any of (i) - (iii); and a pH greater than 8. Preferably the calcium carbonate has particle size distribution of D98 from 40 to 46 microns.

While the specification concludes with claims that particularly point out and distinctly claim the invention, it is believed the present invention will be better understood from the following description.

### DETAILED DESCRIPTION OF THE INVENTION

### Definitions

The term "comprising" as used herein means that steps and ingredients other than those specifically mentioned can be added. This term encompasses the terms "consisting of' and "consisting essentially of." The compositions of the present invention can comprise, consist of, and consist essentially of the essential elements and limitations of the invention described herein, as well as any of the additional or optional ingredients, components, steps, or limitations described herein.

The term "dentifrice" as used herein means paste, gel, powder, tablets, or liquid formulations, unless otherwise specified, that are used to clean the surfaces of the oral cavity. Preferably the dentifrice compositions of the present invention are single phase compositions. The term "teeth" as used herein refers to natural teeth as well as artificial teeth or dental prosthesis.

All percentages, parts and ratios are based upon the total weight of the compositions of the present invention, unless otherwise specified. All such weights as they pertain to listed ingredients are based on the active level and, therefore do not include solvents or by-products that may be included in commercially available materials, unless otherwise specified. The term "weight percent" may be denoted as "wt%" herein.

As used herein, the articles including "a" and "an" when used in a claim, are understood to mean one or more of what is claimed or described.

As used herein, the terms "comprise", "comprises", "comprising", "include", "includes", "including", "contain", "contains", and "containing" are meant to be non-limiting, i.e., other steps and other sections which do not affect the end of result can be added. The above terms encompass the terms "consisting of' and "consisting essentially of'.

As used herein, the words "preferred", "preferably" and variants refer to embodiments of the invention that afford certain benefits, under certain circumstances. However, other embodiments may also be preferred, under the same or other circumstances. Furthermore, the recitation of one or more preferred embodiments does not imply that other embodiments are not useful, and is not intended to exclude other embodiments from the scope of the invention.

### Calcium-containing abrasive

The compositions of the present invention comprise from 25% to 50% by weight of a calcium-containing abrasive, wherein the calcium-containing abrasive is calcium carbonate. Preferably the calcium carbonate is 27% to 47%, preferably 27% to 37%, more preferably from 28% to 34%, yet still more preferably from 29% to 33%, by weight of the composition. More preferably, the calcium-containing abrasive is selected from the group consisting of ground calcium carbonate, precipitated calcium carbonate, and combinations thereof. Fine ground natural chalk (FGNC) is one of the more preferred calcium-containing abrasives useful in the present invention. It is obtained from limestone or marble. FGNC may also be modified chemically or physically by coating during milling or after milling by heat treatment. Typical coating materials include magnesium stearate or oleate. The morphology of FGNC may also be modified during the milling process by using different milling techniques, for example, ball milling, air-classifier milling or spiral jet milling.

Particle size distribution of the calcium carbonate is a critical aspect of the present invention. The present invention is based, in part, on the surprising observation that in high water and high carbonate dentifrice formulations, calcium carbonate particles size has an important impact in fluoride stability. Particle size distribution (in microns) can be characterized by D98, D90, D50, and/or D10. The D50, the median, has been defined above as the diameter where half of the population lies below this value. Similarly, 98 percent of the distribution lies below the D98, 90 percent of the distribution lies below the D90, and 10 percent of the population lies below the D10.

Particle size characterization tools are well known including those based laser diffraction, dynamic light scattering, and dynamic image analysis. One suitable instrument is using a laser diffraction particle sizing instrument MASTERSIZER^{™} 2000 from MALVERN INSTRUMENTS using the methodology described in ISO 13320-1-1999.

One aspect of the invention provides a dentifrice composition wherein the calcium carbonate has a particle distribution size (microns) of D98 from 35 to 46, preferably from 40 to 46, as measured in accordance to ISO 13320-1-1999. More preferably, the calcium carbonate has particle size distribution (microns) of D50 greater than 6.0, preferably from 6.1 to 15, more preferably from 7 to 14, yet more preferably from 10 to 13, as measured in accordance to ISO 13320-1-1999. Yet more preferably, the calcium carbonate has particle size distribution (microns) of D10 greater than 0.7, preferably from 0.8 to 2.5, more preferably from 0.9 to 2.4, yet more preferably from 1 to 2.3, yet still more preferably from 1.5 to 2.1, as measured in accordance to ISO 13320-1-1999.

### Water

The dentifrice compositions of the present invention comprise herein from 45% to 55%, preferably from 46% to 54%, by weight of the composition, water. The water may be added to the formulation and/or may come into the composition from the inclusion of other ingredients. Preferably the water is USP water.

### Fluoride ion source

The compositions include an effective amount of an anti-caries agent. The anti-caries agent is a fluoride ion source. The fluoride ion is present in an amount sufficient to give a fluoride ion concentration in the composition at 25° C, and is used at levels of from 0.0025% to 2% by weight of the composition, alternatively from 0.005% to 2.0% by weight of the composition, to provide anti-caries effectiveness. Representative fluoride ion sources include: stannous fluoride, sodium fluoride, potassium fluoride, amine fluoride, sodium monofluorophosphate, and zinc fluoride. In one embodiment the dentifrice composition contains a fluoride source selected from stannous fluoride, sodium fluoride, and mixtures thereof. In one embodiment, the fluoride ion source is sodium monofluorophosphate, and wherein the composition comprises 0.0025% to 2%, by weight of the composition, of the sodium monofluorophosphate, alternatively from 0.5% to 1.5%, alternatively from 0.6% to 1.7%, alternatively combinations thereof. In one example, the dentifrice compositions of the present invention may have a dual fluoride ion source, specifically sodium monofluorophosphate and an alkaline metal fluoride. Such an approach may provide an improvement in mean fluoride update.

### pH

The pH of the dentifrice composition is greater than pH 8.0.

Preferably the pH is greater than 8.1, more preferably the pH is greater than pH 8.5, even more preferably the pH is greater than pH 9, alternatively the pH is from pH 9.0 to pH 10.5, alternatively from pH 9 to pH 10. The relatively high pH of the present inventive composition may help fluoride stability. Without wishing to be bound theory, at below pH 8 calcium ion may bind with the fluoride. Thus it is desirable to have the dentifrice composition have a greater than pH 8.0 to maximize the stability of the fluoride ion source. A method for assessing pH of dentifrice is described is provided in the analytical methods section provided below. For purposes of clarification, although the analytical method describes testing the dentifrice composition when freshly prepared, for purposes of claiming the present invention, the pH may be taken at anytime during the product's reasonable lifecycle (including but not limited to the time the product is purchased from a store and brought to the consumer's home).

### pH modifying agent

The dentifrice compositions herein may include an effective amount of a pH modifying agent, alternatively wherein the pH modifying agent is a pH buffering agent. pH modifying agents, as used herein, refer to agents that can be used to adjust the pH of the dentifrice compositions to the above-identified pH range. pH modifying agents may include alkali metal hydroxides, ammonium hydroxide, organic ammonium compounds, carbonates, sesquicarbonates, borates, silicates, phosphates, imidazole, and mixtures thereof. Specific pH agents include monosodium phosphate (monobasic sodium phosphate or "MSP"), trisodium phosphate (sodium phosphate tribasic dodecahydrate or "TSP"), sodium benzoate, benzoic acid, sodium hydroxide, potassium hydroxide, imidazole, sodium gluconate, lactic acid, sodium lactate, citric acid, sodium citrate, phosphoric acid. In one embodiment, 0.01% to 3%, preferably from 0.1% to 1%, by weight of the composition, of TSP, and 0.001% to 2%, preferably from 0.01% to 0.3%, by weight of the composition, of monosodium phosphate is used. Without wishing to be bound by theory, TSP and monosodium phosphate may also have calcium ion chelating activity and therefore provide some monofluorophosphate stabilization (in those formulations containing monofluorophosphate).

### Thickening System

The dentifrice compositions of the present invention comprises a thickening system. Preferably the dentifrice composition comprises from 0.5% to 4%, preferably from 0.8% to 3.5%, more preferably from 1% to 3%, yet still more preferably from 1.3% to 2.6%, by weight of the composition, of the thickening system. The thickening system comprising at least a thickening polymer selected from the group consisting of: (a) 0.01% to 3% of a carboxymethyl cellulose ("CMC") by weight of the composition, preferably 0.1% to 2.5%, more preferably 0.2% to 1.5%, by weight of the composition, of CMC; (b) 0.01% to 2.5%, preferably 0.05 % to 2%, more preferably 0.1% to 1.5%, by weight of the composition, of a linear sulfated polysaccharide, preferably wherein the linear sulfated polysaccharide is a carrageenan; (c) 0.01% to 7%, preferably 0.1% to 4%, more preferably from 0.1 % to 2%, yet more preferably from 0.2% to 1.8%, by weight of the composition, of a natural gum, wherein the natural gum is selected from the group consisting of gum karaya, gum arabic, gum tragacanth, xanthan gum; and (d) a combination of any of (a)-(c). The thickening system can comprise a thickening silica, the thickening silica is from 0.01% to 10%, more preferably from 0.1% to 9%, yet more preferably 1% to 8% by weight of the composition.

Preferably the linear sulfated polysaccharide is a carrageenan (also known as carrageenin). Examples of carrageenan include Kappa-carrageenan, Iota-carrageenan, Lambda-carrageenan, and combinations thereof.

In one example the thickening silica is obtained from sodium silicate solution by destabilizing with acid as to yield very fine particles. One commercially available example is ZEODENT^{®} branded silicas from Huber Engineered Materials (e.g., ZEODENT^{®} 103, 124, 113 115, 163, 165, 167).

In one example the CMC is prepared from cellulose by treatment with alkali and monochloro-acetic acid or its sodium salt. Different varieties are commercially characterized by viscosity. One commercially available example is AqualonTM branded CMC from Ashland Special Ingredients (e.g., AqualonTM 7H3SF; AqualonTM 9M3SF AqualonTM TM9A; AqualonTM TM12A).

The natural gum is selected from the group consisting of gum karaya, gum arabic (also known as acacia gum), gum tragacanth, xanthan gum, and combination thereof. More preferably the natural gum is xanthan gum. Xanthan gum is a polysaccharide secreted by the bacterium Xanthomonas camestris. Generally, xanthan gum is composed of a pentasaccharide repeat units, comprising glucose, mannose, and glucuronic acid in a molar ratio of 2:2:1, respectively. The chemical formula (of the monomer) is C₃₅H₄₉O₂₉. In one example, the xanthan gum is from CP Kelco Inc (Okmulgee, US).

### PEG

The compositions of the present invention may comprise polyethylene glycol (PEG), of various weight percentages of the composition as well as various ranges of average molecular weights. In one aspect of the invention, the compositions have from 0.01% to 8%, preferably from 0.1% to 5%, more preferably from 0.2% to 4.8%, yet more preferably from 0.3% to 4.2%, yet still more preferably from 0.5% to 4%, by weight of the composition, of PEG. In another aspect of the invention, the PEG is one having a range of average molecular weight from 100 Daltons to 1600 Daltons, preferably from 200 to 1000, alternatively from 400 to 800, alternatively from 500 to 700 Daltons, alternatively combinations thereof. PEG is a water soluble linear polymer formed by the addition reaction of ethylene oxide to an ethylene glycol equivalent having the general formula: H-(OCH₂CH₂)ₙ-OH. One supplier of PEG is Dow Chemical Company under the brandname of CARBOWAX^{™}. Without wishing to be bound by theory, having some PEG in the dentifrice composition may help with physical stability.

### Anti-calculus agent

The dentifrice compositions may include an effective amount of an anti-calculus agent, which in one embodiment may be present from 0.05% to 50%, by weight of the composition, alternatively from 0.05% to 25%, alternatively from 0.1% to 15% by weight of the composition. Non-limiting examples include those described in US 2011/0104081 A1 at paragraph 64, and those described in US 2012/0014883 A1 at paragraphs 63 to 68, as well as the references cited therein. One example is a pyrophosphate salt as a source of pyrophosphate ion. In one embodiment, the composition comprises tetrasodium pyrophosphate (TSPP) or disodium pyrophosphate or combinations thereof, preferably 0.01% to 2%, more preferably from 0.1% to 1%, by weight of the composition, of the pyrophosphate salt. Without wishing to be bound by theory, TSPP may provide not only calcium chelating thereby mitigating plaque formation, but may also provide the additional benefit of monofluorophosphate stabilization (in those formulations containing monofluorophosphate).

### Surfactant

The dentifrice compositions herein may include a surfactant. The surfactant may be selected from anionic, nonionic, amphoteric, zwitterionic, cationic surfactants, or mixtures thereof. The composition may include a surfactant at a level of from 0.1% to 10%, from 0.025% to 9%, from 0.05% to 5%, from 0.1% to 2.5%, from 0.5% to 2%, or from 0.1% to 1% by weight of the total composition. Non-limiting examples of anionic surfactants may include those described at US 2012/0082630 A1 at paragraphs 32, 33, 34, and 35. Non-limiting examples of zwitterionic or amphoteric surfactants may include those described at US 2012/0082630 A1 at paragraph 36; cationic surfactants may include those described at paragraphs 37 of the reference; and nonionic surfactants may include those described at paragraph 38 of the reference. In one embodiment the composition comprises 0.1% to 5%, preferably 0.1% to 3%, alternatively from 0.3% to 3%, alternatively from 1.2% to 2.4%, alternatively from 1.2% to 1.8%, alternatively from 1.5 % to 1.8%, by weight of the composition, alternatively combinations thereof, of the anionic surfactant sodium lauryl sulfate (SLS).

### Low or Free Humectants

The compositions herein may be substantially free or free of humectants, alternatively contain low levels of humectants. The term "humectant," for the purposes of present invention, include edible polyhydric alcohols such as glycerin, sorbitol, xylitol, butylene glycol, propylene glycol, and combinations thereof. In one embodiment, the humectant is a polyol, preferably wherein the polyol is selected from sorbitol, glycerin, and combinations thereof. In yet another embodiment, the humectant is sorbitol. In one embodiment, the composition comprises from 0% to less than 5%, by weight of the composition, of humectants, preferably from 0% to 4%, alternatively from 0% to 3%, alternatively from 0% to 2%, alternatively from 0% to 1%, by weight of th4 composition, of humectants. A potential advantage of having a dentifrice composition that is free or substantially free of humectants is, without wishing to be bound by theory, is those dentifrice compositions that are free of polyols (e.g., glycerin and sorbitol), or have a relatively low amount thereof, may provide better fluoride uptake compared to those compositions having the high levels of such polyols (or humectants for that matter). Preferably, the dentifrice compositions of the present invention comprise from 0% to 5%, preferably 0% to 3%, more preferably 0% to 1%, by weight of the composition, of glycerin, sorbitol, or combinations thereof; yet more preferably the composition is substantially free of both glycerin and sorbitol.

### Sweetener

The oral care compositions herein may include a sweetening agent. These sweetener agents may include saccharin, dextrose, sucrose, lactose, maltose, levulose, aspartame, sodium cyclamate, D-tryptophan, dihydrochalcones, acesulfame, sucralose, neotame, and mixtures thereof. Sweetening agents are generally used in oral compositions at levels of from 0.005% to 5%, by weight of the composition, alternatively 0.01% to 1%, alternatively from 0.1% to 0.5%, alternatively combinations thereof.

### Colorant

The compositions herein may include a colorant. Titanium dioxide is one example of a colorant. Titanium dioxide is a white powder which adds opacity to the compositions. Titanium dioxide generally can comprise from 0.25% to 5%, by weight of the composition.

### Flavorant

The compositions herein may include from 0.001% to about 5%, alternatively from 0.01% to 4%, alternatively from 0.1% to 3%, alternatively from 0.5% to 2%, alternatively 1% to 1.5%, alternatively 0.5% to 1%, by weight of the composition, alternatively combinations thereof, of a flavorant composition. The term flavorant composition is used in the broadest sense to include flavor ingredients, or sensates, or sensate agents, or combinations thereof. Flavor ingredients may include those described in US 2012/0082630 A1 at paragraph 39; and sensates and sensate ingredients may include those described at paragraphs 40 - 45. Excluded from the definition of flavorant composition is "sweetener" (as described above).

### Viscosity

A viscosity of 150000 cP to 850000 cP is a classic viscosity target range for a consumer acceptable dentifrice. The compositions of the present invention are preferably within this range. A method for assessing viscosity is described. The viscometer is Brookfield^{®} viscometer, Model DV-I Prime with a Brookfield "Helipath" stand. The viscometer is placed on the Helipath stand and leveled via spirit levels. The E spindle is attached, and the viscometer is set to 2.5 RPM. Detach the spindle, zero the viscometer and install the E spindle. Then, lower the spindle until the crosspiece is partially submerged in the paste before starting the measurement. Simultaneously turn on the power switch on the viscometer and the helipath to start rotation of the spindle downward. Set a timer for 48 seconds and turn the timer on at the same time as the motor and helipath. Take a reading after the 48 seconds. The reading is in cP.

### Phase Stability

The term "phase stability" means visually (i.e., to the unaided eye) having no liquid separated from the oral care composition (e.g., toothpaste) body over a defined period of time under ambient conditions. In other words, a phase stable composition will resist syneresis. The compositions of the present invention are preferably phase stable for at least 6 months, more preferably 12 months or more.

### EXAMPLES

### Analytical Methods

The method for assessing soluble fluoride is described consistent with the China's National Standard Method GB8372-2008. Briefly, an ion-selective electrode (ISE) is used to test soluble fluoride in dentifrice. An example of a fluoride ion meter is SARTORIUS PP-50, but an equivalent may be used. The ion meter may be fitted with a fluoride-specific ion electrode with a single-junction reference electrode by Orion Research Inc., Cat. No. 9609BNWP, but an equivalent may be used. The sample is prepared by using a balance that is accurate to the 0.0001 gram (g). 20 g of dentifrice is weighed into a tarred 50 mL plastic beaker and then gradually 50mL of deionized water is added, while a magnetic stir bar is stirring in the plastic beaker, until the dentifrice is a completely disperse solution. The entire solution is gently transferred to a 100mL plastic volumetric flask as to avoid generating foam (so the volume can be measured accurately), and deionized water is added to reach a total volume 100ml, and then the solution is shaken manually to form a slurry. The formed slurry is then transferred into 10 mL centrifuge tubes, and centrifued for 10 minutes at 15000 rotations-per-minute (RPM) (at 24149g force) at ambient temperature. Thereafter 0.5 mL of supernatant is transferred into a 2 mL mini-centrifugal tube, and 0.7 mL of 4 mol/L HCl is added to the tub. Then the tub is capped, heated in a 50° C waterbath for 10 minutes. Thereafter the contents of the tub are transferred to a 50 mL measuring flask. The following are also added to the flask: 0.7 mL of 4 mol/L NaOH to neutralize the solution; 5 mL of citrate buffer solution (described further below); deionzed water is added until a total volume of 50 mL is achieved in the flask; and then the sample solution is gently mixed. The aforementioned citrate buffer solution is prepared by dissolving 100 g of sodium citrate, 60 mL of glacial acetic acid, 60 g of NaCl, and 30g of NaOH, all with water, adjusting the pH to 5.0~5.5, and diluting the citrate buffer solution with deionized water until a total volume of 1000 mL is achieved. Turning back to the sample solution, the entire 50 mL solution is transferred to a 50 mL plastic beaker and the fluoride level is assessed based on a fluoride standard curve using the fluoride ion meter and electrode described.

The standard fluoride curve (w/w %) is prepared by accurately measuring 0.5 mL, 1.0 mL, 1.5 mL, 2.0 mL, and 2.5 mL fluoride ion standard solutions (100 mg/kg) into five respective 50 mL plastic measuring flasks. 5mL of citrate buffer solution (made as previously described above) into each respective flask, and then diluting each solution to the scale with deionized water. Thereafter, each solution is transferred into a 50 mL plastic beaker respectively, measuring potential E under magnetic agitation, recording potential values, and drawing *E*-log*c* (wherein "c" is a concentration) standard curve.

A method for assessing pH of dentifrice is described. pH is measured by a pH Meter with Automatic Temperature Compensating (ATC) probe. The pH Meter is capable of reading to 0.001 pH unit. The pH electrode may be selected from one of the following (i) Orion Ross Sure-Flow combination: Glass body - VWR #34104-834/Orion #8172BN or VWR#10010-772/Orion #8172BNWP; Epoxy body - VWR #34104-830/Orion #8165BN or VWR#10010-770/Orion #8165BNWP; Semi-micro, epoxy body - VWR #34104-837/Orion #8175BN or VWR#10010-774/Orion #3175BNWP; or (ii) Orion PerpHect combination:VWR #34104-843/Orion #8203BN semi-micro, glass body; or (iii) suitable equivalent. The automatic temperature compensating probe is Fisher Scientific, Cat #13-620-16.

A 25% by weight slurry of dentifrice is prepared with deionized water, and thereafter is centrifuged for 10 minutes at 15000 rotations-per-minute using a SORVALL RC 28S centrifuge and SS-34 rotor (or equivalent gravitational force, at 24149g force). The pH is assessed in supernatant after one minute. After each pH assessment, the electrode is washed with deionized water. Any excess water is wiped with a laboratory grade tissue. When not in issue, the electrode is kept immersed in a pH 7 buffer solution or an appropriate electrode storage solution.

### Compositional Components

**Table 1: Compositional components of inventive example 2 and comparative examples 1 and 3 are provided:**

| Components: (Wt%) | Ex. 1 Comparative | Ex. 2 Inventive | Ex. 3 Comparative |
|---|---|---|---|
| CaCO₃ (325 Mesh) | 0 | 32.00 | 0 |
| CaCO₃ (600 Mesh) | 32.00 | 0 | 42.00 |
| Water | 55.52 | 55.52 | 45.52 |
| Sodium Monofluorophosphate ("Na-MFP") | 1.10 | 1.10 | 1.10 |
| Sodium Caboxy-methyl Cellulose | 0.91 | 0.91 | 0.91 |
| Carrageenan | 1.41 | 1.41 | 1.41 |
| Thickener Silica | 2.62 | 2.62 | 2.62 |
| Sodium Lauryl Sulfate | 4.00 | 4.00 | 4.00 |
| Tetra Sodium Pyrophosphate | 0.42 | 0.42 | 0.42 |
| Flavor | 0.85 | 0.85 | 0.85 |
| Sodium Monophosphate | 0.08 | 0.08 | 0.08 |
| Sodium Triphosphate | 0.42 | 0.42 | 0.42 |
| Sodium Saccharine | 0.58 | 0.58 | 0.58 |
| Total: | 100 | 100 | 100 |
| Initial pH: | 9.39 | 9.39 | 9.34 |

Referring to Table 1, inventive example 2 differs from comparative examples 1 and 3 in at least one fundamental way, which is the relative size of the carbonate particles. The inventive composition notably contains a relatively larger calcium carbonate Mesh size 325 compared to the comparative examples 1 and 3 (having the smaller particle size of Mesh size 600). As between the comparative examples, example 3 contains more of the calcium carbonate (at 42 wt%) compared to example 1 (at 32 wt%). Particle size distribution is a more precise way of characterizing Mesh size.

### Data

Fluoride stability and pH change of the three examples are provided in Table 2. Examples 1 and 3 are comparative examples, while example 2 is an inventive composition. The compositional components of these examples are described in earlier Table 1.

**Table 2a: Fluoride Stability Profile at 30° C Two years**

| Weeks: | Examples: | | | | | |
|---|---|---|---|---|---|---|
| | Ex. 1 (600M CaCO₃) Comparative | | Ex. 2 (325M CaCO₃) Inventive | | Ex. 3 (600M CaCO₃) Comparative | |
| | Soluble Fluoride (PPM) | pH | Soluble Fluoride (PPM) | pH | Soluble Fluoride (PPM) | pH |
| 0 | 1160 | 9.39 | 1200 | 9.39 | 1138 | 9.34 |
| 26 | 1132 | 8.86 | 1200 | 9.17 | 1000 | 8.92 |
| 52 | 912 | 8.92 | 1200 | 8.99 | 700 | 8.96 |
| 78 | 730 | 8.86 | 1279 | 8.81 | 494 | 8.97 |
| 104 | 592 | 8.91 | 1119 | 8.71 | 412 | 9.07 |

**Table 2b: Fluoride Loss after Two years at 30° C**

| | Ex. 1 (32% 600M CaCO₃) Comparative | Ex. 2 (32% 325M CaCO₃) Inventive | Ex. 3 (42% 600M CaCO₃) Comparative |
|---|---|---|---|
| Total Soluble Fluoride loss^{∗} (PPM) | 568 | 81 | 726 |

| | | | |
|---|---|---|---|
| ^{∗}104 weeks | | | |

There are a number of observations that can be obtained from data of Tables 2a and 2b. Firstly, comparing the two comparative examples (Ex. 1 and 3) to each other, there is a greater drop in fluoride stability over the two years with example 3, i.e., the dentifrice composition containing more of the calcium carbonate. The only difference between examples 1 and 3 is the amount of calcium carbonate (and water). Both examples 1 and 3 have the relatively smaller calcium carbonate particles of Mesh size 600, but example 3 has a greater amount of the calcium carbonate (and less water) as compared to example 1. Over the course of two years, example 3 has soluble fluoride parts per million (PPM) loss of 726 while example 1 has a loss of 568 PPM. Accordingly, and given the greater soluble fluoride loss in example 3, there is a suggestion that a greater amount of calcium carbonate in the subject dentifrice chassis leads to greater fluoride stability loss. This underscored the need for a calcium carbonate type that can minimize or mitigate the loss of soluble fluoride over time in the higher water and high carbonate dentifrice formulation chassis described herein.

Comparing inventive example 2 to comparative example 1, the inventive dentifrice composition has significantly much lower loss of soluble fluoride over time. Over the course of two years, example 2 has soluble fluoride PPM loss of 726 while example 1 has only a loss of 568. The only difference between the examples 1 and 2 is the Mesh size (i.e., particle distribution) of the calcium carbonate particles. Inventive example 2 has the relatively larger particles of calcium carbonate of 325 Mesh size where as the comparative example 1 has the relatively smaller particles of calcium carbonate of 600 Mesh size. Similar results are observed comparing inventive example 2 and comparative example 3. Indeed comparative example 3 had the greatest amount of fluoride loss likely given that it has the most calcium carbonate and of the less desirable smaller Mesh sized calcium carbonate particles.

### Particle Size Distribution of Calcium Carbonate Particles

The particle size of the particulates in the CaCO₃ is measured using a laser diffraction particle sizing instrument (Mastersizer^{™} 2000 from Malvern Instruments). The laser diffraction technique works by measuring the light scattered from particulates as they pass through a laser beam. Particulates scatter light at an angle that is directly related to their size. The Mastersizer^{™} 2000 uses the light scattering pattern associated with a sample to calculate particle size distributions. The instrument follows the recommendations of ISO 13320-1-1999. CaCO₃ raw material is dispersed into deionized water in Mastersizer^{™} 2000 sample beaker at 2000 rpm stirring speed. The dispersion is re-circulated between the beaker and the sampling cell of the particle sizing instrument where the particle size is measured. Particle size distribution parameters D98/D90/D50/D10 are obtained for each sample in the instrument standard software.

**Table 3: Particle Size Parameters of Calcium Carbonate particles of 325 Mesh and 600 Mesh sizes are measured in accordance with ISO13320-1-1999.**

| Particle Size Parameter | CaCO₃ Particle (microns) | |
|---|---|---|
| | Size: 325M | Size: 600M |
| D10 | 2.058 | 0.7 (lower limit) |
| D50 | 12.224 | 3.0 - 6.0 |
| D90 | 31.711 | 7.4 - 15.4 |
| D98 | 45.021 | 26.0 (upper limit) |

As the data in Table 3 indicates, the particles size parameters of 325 Mesh calcium carbonate are much larger than those of 600 Mesh calcium carbonate. The 325Mesh calcium carbonate can be obtained commercially from Guangxi Mantingfang Fine Chemical (Guangxi, China)

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

## Claims

1. A dentifrice composition comprising:
(a) 45% to 55%, by weight of the composition, of water;
(b) 25% to 50%, by weight of the composition, of a calcium carbonate,
wherein the calcium carbonate has a particle distribution size of D98 from 35 microns to 46 microns as measured by laser diffraction particle sizing per method ISO 13320-1-1999;
(c) 0.0025% to 2%, by weight of the composition, of a fluoride ion source;
(d) a thickening system comprising at least a thickening polymer, wherein the thickening polymer is selected from
(i) 0.01% to 3% of a carboxymethyl cellulose (CMC) by weight of the composition;
(ii) 0.01% to 2.5% by weight of the composition of a linear sulfated polysaccharide, and;
(iii) 0.01% to 7% by weight of the composition of a natural gum, wherein the natural gum is selected from the group consisting of gum karaya, gum arabic, gum tragacanth, xanthan gum and
(iv) a combination of any of (i) - (iii); and
a pH greater than 8.

2. The dentifrice composition according to claim 1, wherein the calcium carbonate has particle size distribution of D98 from 40 to 46 microns; and preferably the calcium carbonate has particle size distribution of D90 greater than 15.4 microns, preferably from 15.5 microns to 35 microns, more preferably from 16 to 34 microns, yet more preferably from 20 to 33 microns, yet still more preferably from 25 to 32 microns.

3. The dentifrice composition according to any one of the preceding claims, wherein the calcium carbonate has particle size distribution of D50 greater than 6.0 microns, preferably from 6.1 microns to 15 microns, more preferably from 7 to 14 microns, yet more preferably from 10 to 13 microns.

4. The dentifrice composition according to any one of the preceding claims, wherein the calcium carbonate has particle size distribution of D10 greater than 0.7 microns, preferably from 0.8 microns to 2.5 microns, more preferably from 0.9 to 2.4 microns, yet more preferably from 1 to 2.3 microns, yet still more preferably from 1.5 to 2.1 microns.

5. The dentifrice composition according to any one of the preceding claims, wherein the calcium carbonate is 27% to 47%, preferably 27% to 37%, more preferably from 28% to 34%, by weight of the composition.

6. The dentifrice composition according to any one of the preceding claims, wherein the pH is greater than 8.1, preferably greater than pH 8.5, more preferably at or greater than pH 9, alternatively from pH 9.0 to pH 10.5.

7. The dentifrice composition according to any one of the preceding claims, wherein the fluoride ion source is sodium monofluorophosphate, preferably wherein the sodium monofluorophosphate is from 0.5% to 1.5% by weight of the composition.

8. The dentifrice composition according to any one of the preceding claims, wherein the thickening polymer is selected from group consisting of:
(i) 0.1% to 2.5%, preferably 0.2% to 1.5%, by weight of the composition, of CMC;
(ii) 0.05 % to 2%, preferably 0.1% to 1.5%, by weight of the composition, of a linear sulfated polysaccharide, preferably wherein the linear sulfated polysaccharide is a carrageenan;
(iii) 0.1% to 4%, preferably from 0.1 % to 2%, more preferably from 0.2% to 1.8%, by weight of the composition, of a natural gum.

9. The dentifrice composition according to any one of the preceding claims, wherein the thickening system comprises further a thickening silica, wherein preferably the thickening silica is from 0.01% to 10%, more preferably from 0.1% to 9%, yet more preferably 1% to 8% by weight of the composition.

10. The dentifrice composition according to any one of the preceding claims, wherein the dentifrice comprises from 0.5% to 4%, preferably from 0.8% to 3.5%, more preferably from 1% to 3%, yet still more preferably from 1.3% to 2.6%, by weight of the composition of the thickening system.

11. The dentifrice composition according to any one of the preceding claims, wherein the composition is characterized as having viscosity from 150000 mPa·s (=150000 cP) to 850000 mPa·s (=850000 cP) as measured with a Brookfield^{®} viscometer, Model DV-I Prome with a Brookfield "Helipath" stand according to the method described herein, preferably the dentifrice composition is a single phase composition

12. The dentifrice composition according to any one of the preceding claims, further comprising 0.1% to 12%, by weight of the composition, of a surfactant, preferably 1% to 9% of the surfactant, more preferably wherein the surfactant is an anionic surfactant, yet more preferably wherein the anionic surfactant is sodium lauryl sulfate.

13. The dentifrice composition according to any one of the preceding claims, further comprising a polyethylene glycol ("PEG"), preferably the composition comprises from 0.1% to 5%, by weight of the composition, of the PEG, more preferably from 0.5% to 4%, yet more preferably from 1% to 3%, by weight of the composition, of PEG.

## Patentansprüche

1. Zahncremezusammensetzung, umfassend:
(a) zu 45 Gew.-% bis 55 Gew.-% der Zusammensetzung Wasser;
(b) zu 25 Gew.-% bis 50 Gew.-% der Zusammensetzung Calciumcarbonat;
wobei das Calciumcarbonat eine Partikelverteilungsgröße von D98 von 35 Mikrometer bis 46 Mikrometer aufweist, gemessen durch Messung der Partikelgrößenverteilung mittels Laserbeugung gemäß Verfahren ISO 13320-1-1999;
(c) zu 0,0025 Gew.-% bis 2 Gew.-% der Zusammensetzung eine Fluoridionenquelle;
(d) ein Verdickungssystem, das mindestens ein Verdickungspolymer umfasst, wobei das Verdickungspolymer ausgewählt ist aus
(i) zu 0,01 Gew.-% bis 3 Gew.-% der Zusammensetzung Carboxymethylcellulose (CMC);
(ii) zu 0,01 Gew.-% bis 2,5 Gew.-% der Zusammensetzung ein lineares sulfatiertes Polysaccharid, und;
(iii) zu 0,01 Gew.-% bis 7 Gew.-% der Zusammensetzung einen Naturgummi, wobei das Naturgummi ausgewählt ist aus der Gruppe bestehend aus Karayagummi, Gummiarabikum, Gummitragant, Xanthangummi und
(iv) eine Kombination von einem von (i) - (iii); und
einen pH-Wert, der größer als 8 ist.

2. Zahncremezusammensetzung nach Anspruch 1, wobei das Calciumcarbonat eine Partikelgrößenverteilung von D98 von 40 bis 46 Mikrometer aufweist; und das Calciumcarbonat vorzugsweise eine Partikelgrößenverteilung von D90 von mehr als 15,4 Mikrometer, vorzugsweise von 15,5 Mikrometer bis 35 Mikrometer, mehr bevorzugt von 16 bis 34 Mikrometer, noch mehr bevorzugt von 20 bis 33 Mikrometer, noch mehr bevorzugt von 25 bis 32 Mikrometer aufweist.

3. Zahncremezusammensetzung nach einem der vorstehenden Ansprüche, wobei das Calciumcarbonat eine Partikelgrößenverteilung von D50 von mehr als 6,0 Mikrometer, vorzugsweise von 6,1 Mikrometer bis 15 Mikrometer, mehr bevorzugt von 7 bis 14 Mikrometer, noch mehr bevorzugt von 10 bis 13 Mikrometer aufweist.

4. Zahncremezusammensetzung nach einem der vorstehenden Ansprüche, wobei das Calciumcarbonat eine Partikelgrößenverteilung von D10 von mehr als 0,7 Mikrometer, vorzugsweise von 0,8 Mikrometer bis 2,5 Mikrometer, mehr bevorzugt von 0,9 bis 2,4 Mikrometer, noch mehr bevorzugt von 1 bis 2,3 Mikrometer, noch mehr bevorzugt von 1,5 bis 2,1 Mikrometer aufweist.

5. Zahncremezusammensetzung nach einem der vorstehenden Ansprüche, wobei das Calciumcarbonat 27 Gew.-% bis 47 Gew.-%, vorzugsweise 27 Gew.-% bis 37 Gew.-%, mehr bevorzugt von 28 Gew.-% bis 34 Gew.-% der Zusammensetzung ausmacht.

6. Zahncremezusammensetzung nach einem der vorstehenden Ansprüche, wobei der pH-Wert größer als 8,1 ist, vorzugsweise größer als pH-Wert 8,5, mehr bevorzugt größer als pH-Wert 9, alternativ von pH-Wert 9,0 bis pH-Wert 10,5 beträgt.

7. Zahncremezusammensetzung nach einem der vorstehenden Ansprüche, wobei die Fluoridionenquelle Natriummonofluorphosphat ist, wobei vorzugsweise das Natriummonofluorphosphat von 0,5 Gew.-% bis 1,5 Gew.-% der Zusammensetzung ausmacht.

8. Zahncremezusammensetzung nach einem der vorstehenden Ansprüche, wobei das Verdickungspolymer ausgewählt ist aus der Gruppe bestehend aus:
(i) zu 0,1 Gew.-% bis 2,5 Gew.-%, vorzugsweise zu 0,2 Gew.-% bis 1,5 Gew.-% der Zusammensetzung CMC;
(ii) zu 0,05 Gew.-% bis 2 Gew.-%, vorzugsweise zu 0,1 Gew.-% bis 1,5 Gew.-% der Zusammensetzung einem linearen sulfatierten Polysaccharid, wobei das lineare sulfatierte Polysaccharid vorzugsweise ein Carrageenan ist;
(iii) zu 0,1 Gew.-% bis 4 Gew.-%, vorzugsweise zu 0,1 Gew.-% bis 2 Gew.-%, mehr bevorzugt zu 0,2 Gew.-% bis 1,8 Gew.-% der Zusammensetzung einem Naturgummi.

9. Zahncremezusammensetzung nach einem der vorstehenden Ansprüche, wobei das Verdickungssystem ferner ein Verdickungssiliciumdioxid umfasst, wobei das Verdickungssiliciumdioxid vorzugsweise von 0,01 Gew.-% bis 10 Gew.-%, mehr bevorzugt von 0,1 Gew.-% bis 9 Gew.-%, noch mehr bevorzugt 1 Gew.-% bis 8 Gew.-% der Zusammensetzung beträgt.

10. Zahncremezusammensetzung nach einem der der vorstehenden Ansprüche wobei die Zahncreme zu 0,5 Gew.-% bis 4 Gew.-%, vorzugsweise zu 0,8 Gew.-% bis 3,5 Gew.-%, mehr bevorzugt zu 1 Gew.-% bis 3 Gew.-%, noch mehr bevorzugt zu 1,3 Gew.-% bis 2,6 Gew.-% der Zusammensetzung das Verdickungssystem umfasst.

11. Zahncremezusammensetzung nach einem der vorstehenden Ansprüche, wobei die Zusammensetzung **dadurch gekennzeichnet ist, dass** sie eine Viskosität von 150000 mPa·s (=150000 cP) bis 850000 mPa·s (=850000 cP) aufweist, gemessen mit einem Brookfield^{®}-Viskosimeter, Modell DV-I Prime mit einem Brookfield "Helipath" gemäß dem hierin beschriebenen Verfahren, vorzugsweise ist die Zahncremezusammensetzung eine einphasige Zusammensetzung.

12. Zahncremezusammensetzung nach einem der vorstehenden Ansprüche, ferner umfassend zu 0,1 Gew.-% bis 12 Gew.-% der Zusammensetzung ein Tensid, vorzugsweise zu 1 Gew.-% bis 9 Gew.-% das Tensid, wobei das Tensid mehr bevorzugt ein anionisches Tensid ist, wobei das anionische Tensid noch mehr bevorzugt ein Natriumlaurylsulfat ist.

13. Zahncremezusammensetzung nach einem der vorstehenden Ansprüche, ferner umfassend Polyethylenglykol ("PEG"), wobei die Zusammensetzung vorzugsweise zu 0,1 Gew.-% bis 5 Gew.-% der Zusammensetzung das PEG, mehr bevorzugt zu 0,5 Gew.-% bis 4 Gew.-%, noch mehr bevorzugt zu 1 Gew.-% bis 3 Gew.-% der Zusammensetzung PEG umfasst.

## Revendications

1. Composition dentifrice comprenant :
(a) 45 % à 55 %, en poids de la composition, d'eau ;
(b) 25 % à 50 %, en poids de la composition, d'un carbonate de calcium,
dans laquelle le carbonate de calcium a une taille granulométrique de D98 allant de 35 micromètres à 46 micromètres telle que mesurée par granulométrie à diffraction laser selon le procédé ISO 13320-1-1999 ;
(c) 0,0025 % à 2 %, en poids de la composition, d'une source d'ions fluorure ;
(d) un système épaississant comprenant au moins un polymère épaississant, dans laquelle le polymère épaississant est choisi parmi
(i) 0,01 % à 3 % d'une carboxyméthylcellulose (CMC) en poids de la composition ;
(ii) 0,01 % à 2,5 % en poids de la composition d'un polysaccharide sulfaté linéaire, et ;
(iii) 0,01 % à 7 % en poids de la composition d'une gomme naturelle, dans laquelle la gomme naturelle est choisie dans le groupe constitué de gomme de karaya, gomme arabique, gomme adragante, gomme de xanthane et
(iv) un combinaison de n'importe lesquels parmi (i) - (iii) ; et
un pH supérieur à 8.

2. Composition dentifrice selon la revendication 1, dans laquelle le carbonate de calcium a une distribution granulométrique de D98 allant de 40 à 46 micromètres ; et de préférence le carbonate de calcium a une distribution granulométrique de D90 supérieure à 15,4 micromètres, de préférence de 15,5 micromètres à 35 micromètres, plus préférablement de 16 à 34 micromètres, encore plus préférablement de 20 à 33 micromètres, encore même plus préférablement de 25 à 32 micromètres.

3. Composition dentifrice selon l'une quelconque des revendications précédentes, dans laquelle le carbonate de calcium a une distribution granulométrique de D50 supérieure à 6,0 micromètres, de préférence de 6,1 micromètres à 15 micromètres, plus préférablement de 7 à 14 micromètres, encore plus préférablement de 10 à 13 micromètres.

4. Composition dentifrice selon l'une quelconque des revendications précédentes, dans laquelle le carbonate de calcium a une distribution granulométrique de D10 supérieure à 0,7 micromètre, de préférence de 0,8 micromètre à 2,5 micromètres, plus préférablement de 0,9 à 2,4 micromètres, encore plus préférablement de 1 à 2,3 micromètres, encore même plus préférablement de 1,5 à 2,1 micromètres.

5. Composition dentifrice selon l'une quelconque des revendications précédentes, dans laquelle le carbonate de calcium représente 27 % à 47 %, de préférence 27 % à 37 %, plus préférablement de 28 % à 34 %, en poids de la composition.

6. Composition dentifrice selon l'une quelconque des revendications précédentes, dans laquelle le pH est supérieur à 8,1, de préférence supérieur à pH 8,5, plus préférablement égal ou supérieur à pH 9, en variante de pH 9,0 à pH 10,5.

7. Composition dentifrice selon l'une quelconque des revendications précédentes, dans laquelle la source d'ions fluorure est du monofluorophosphate de sodium, de préférence dans laquelle le monofluorophosphate de sodium représente de 0,5 % à 1,5 % en poids de la composition.

8. Composition dentifrice selon l'une quelconque des revendications précédentes, dans laquelle le polymère épaississant est choisi dans le groupe constitué de :
(i) 0,1 % à 2,5 %, de préférence 0,2 % à 1,5 %, en poids de la composition, de CMC ;
(ii) 0,05 % à 2 %, de préférence 0,1 % à 1,5 %, en poids de la composition, d'un polysaccharide sulfaté linéaire, de préférence dans laquelle le polysaccharide sulfaté linéaire est du carraghénane ;
(iii) 0,1 % à 4 %, de préférence de 0,1 % à 2 %, plus préférablement de 0,2 % à 1,8 %, en poids de la composition, d'une gomme naturelle.

9. Composition dentifrice selon l'une quelconque des revendications précédentes, dans laquelle le système épaississant comprend en outre une silice épaississante, dans laquelle de préférence la silice épaississante représente de 0,01 % à 10 %, plus préférablement de 0,1 % à 9 %, encore plus préférablement 1 % à 8 % en poids de la composition.

10. Composition dentifrice selon l'une quelconque des revendications précédentes, dans laquelle le dentifrice comprend de 0,5 % à 4 %, de préférence de 0,8 % à 3,5 %, plus préférablement de 1 % à 3 %, encore même plus préférablement de 1,3 % à 2,6 %, en poids de la composition du système épaississant.

11. Composition dentifrice selon l'une quelconque des revendications précédentes, dans laquelle la composition est **caractérisée en ce qu'**elle a une viscosité allant de 150 000 mPa·s (=150 000 cP) à 850 000 mPa·s (=850 000 cP) telle que mesurée avec un viscosimètre Brookfield^{®}, Modèle DV-I prime avec un support Brookfield « Helipath » selon le procédé décrit ici, de préférence la composition dentifrice est une composition monophasique.

12. Composition dentifrice selon l'une quelconque des revendications précédentes, comprenant en outre 0,1 % à 12 % d'un agent tensioactif en poids de la composition, de préférence 1 % à 9 % de l'agent tensioactif, plus préférablement dans laquelle l'agent tensioactif est un agent tensioactif anionique, encore plus préférablement dans laquelle l'agent tensioactif anionique est du laurylsulfate de sodium.

13. Composition dentifrice selon l'une quelconque des revendications précédentes, comprenant en outre un polyéthylène glycol (« PEG »), de préférence la composition comprend de 0,1 % à 5 %, en poids de la composition, du PEG, plus préférablement de 0,5 % à 4 %, encore plus préférablement de 1 % à 3 %, en poids de la composition, de PEG.
